(19) Europäisches Patentamt
European Patent Office
Office européen des brevets

(11) EP 3 866 750 B1

(12) **EUROPEAN PATENT SPECIFICATION**

(45) Date of publication and mention of the grant of the patent:
**18.06.2025 Bulletin 2025/25**

(21) Application number: **19873528.4**

(22) Date of filing: **16.10.2019**

(51) International Patent Classification (IPC):
***A61K 8/04** (2006.01)* ***A61P 31/00** (2006.01)*
***A61K 8/06** (2006.01)* ***A61K 8/11** (2006.01)*
***A61K 31/14** (2006.01)* ***A01N 25/04** (2006.01)*

(52) Cooperative Patent Classification (CPC):
(C-Sets available)
**A61Q 11/00; A01N 25/04; A01P 1/00; A61K 8/044; A61K 8/26; A61K 8/345; A61K 8/416; A61K 9/0019; A61K 31/14; A61K 45/06; A61K 47/02; A61K 47/10; A61P 31/00** (Cont.)

(86) International application number:
**PCT/US2019/056429**

(87) International publication number:
**WO 2020/081624 (23.04.2020 Gazette 2020/17)**

(54) **SUSPENSION FORMULATIONS OF HIGH LOAD DISPERSIONS**
SUSPENSIONSFORMULIERUNGEN VON DISPERSIONEN MIT HOHER LAST
FORMULATIONS DE SUSPENSION DE DISPERSIONS À CHARGE ÉLEVÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2018 US 201862746608 P**

(43) Date of publication of application:
**25.08.2021 Bulletin 2021/34**

(73) Proprietor: **Austin Research Labs Corp.**
**Buffalo, New York 14210 (US)**

(72) Inventors:
- **SUPAMAHITORN, Jai**
  **BUFFALO,, New York 14210 (US)**
- **MCMILLAN, Lauren**
  **BUFFALO, New York 14210 (US)**

(74) Representative: **Algemeen Octrooi- en Merkenbureau B.V.**
**P.O. Box 645**
**5600 AP Eindhoven (NL)**

(56) References cited:
**WO-A1-2012/054090** **WO-A1-98/24791**
**US-A- 5 015 469** **US-A1- 2003 161 866**
**US-A1- 2006 045 868** **US-A1- 2016 000 920**
**US-A1- 2017 354 733** **US-B1- 6 551 607**
**US-B2- 8 858 970**

- **DENNIS LABA: "How do I thicken my cosmetic formula", COSMETICS ANS TOILETRIES, vol. 116, no. 11, 30 November 2001 (2001-11-30), pages 35 - 44, XP055301132**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

(Cont. next page)

(52) Cooperative Patent Classification (CPC): (Cont.)

C-Sets
**A01N 25/04, A01N 33/12, A01N 39/02;**
**A61K 31/14, A61K 2300/00**

## Description

### CROSS-REFERENCE TO RELATED APPLICATION

This application claims priority to United States provisional patent application serial number 62/746,608, filed October 17, 2018.

### FIELD OF THE DISCLOSURE

The present disclosure relates to the field of carrier and delivery systems for active molecular compounds. In particular, the present disclosure provides suspension formulations of aqueous dispersions for delivery to subjects (e.g., by injection).

### BACKGROUND OF THE DISCLOSURE

Sepsis affects more than 750,000 patients annually in the United States and has a mortality rate of from 30 to 65%, which makes it the tenth most common cause of death in the U.S. The risk of sepsis is found to be inversely related to age. Sepsis accounts for 60 to 80% of childhood deaths in the developing world. As a result of its prevalence, hospital visits for sepsis or septicemia increased from 621,000 in the year 2000 to 1,141,000 in 2008. In economic terms, total costs for treating sepsis increased by an average of 11.9% each year between 1997 and 2008, adjusted for inflation, and amounted to $14.6 billion in the U.S. in 2008 and currently exceed $17 billion. In addition to other hospital treatments, over 2.5 million patients are admitted annually to ICUs for sepsis with the costs per individual ICU case adding $5,000 or more per day to total hospital costs and with treatment lasting at least two days and often more than 20 days. Additionally, nearly 50 percent of diagnosed sepsis cases in the U.S. are attributable to hospital-acquired infections (HAIs), which impose a major direct cost on hospitals due to little to no reimbursement from health insurers.

Sepsis is the body's response to infection. This response is characterized by the cardinal signs of inflammation (e.g., vasodilation, leukocyte accumulation, and increased microvascular permeability) occurring in tissues that are remote from the infection. Even a minor infection, such as strep throat or influenza, can trigger sepsis. Sepsis is usually not life-threatening, but complications of sepsis can cause serious illness and death. Sepsis is a general term describing immune responses within a continuum from infection to multiple organ dysfunction syndrome. Systemic inflammatory response syndrome (SIRS) is the presence of two or more of abnormal body temperature, heart rate, respiratory rate or blood gas, and white blood cell count, and sepsis is defined as SIRS in response to an infectious process. Severe sepsis is defined as sepsis with sepsis-induced organ dysfunction or tissue hypoperfusion (e.g., manifesting as hypotension, elevated lactate, or decreased urine output). Severe sepsis occurs when a natural immune response to an infection triggers widespread inflammation and blood clotting in tiny vessels throughout the body, which also involves failure of critical organs in the body and can thus lead to death. Finally, septic shock is severe sepsis plus persistently low blood pressure.

While the existing technologies for treating sepsis have increased success rates of therapies, additional therapies are needed.

US 2016/000920 A1 discloses a composition comprising a suspension of a dispersion in glycerine (moisturiser or humectant), comprising a) a layered hydrous magnesium silicate having binding sites in said dispersed phase; and b) a quaternary ammonium compound in said dispersed phase; wherein said quaternary ammonium compound is loaded into said layered hydrous magnesium silicate clay with a percentage loading relative to available layered hydrous magnesium silicate clay binding sites of greater than 200%. US 2016/000920 further discloses that the quaternary ammonium compound comprises benzethonium chloride. US 2016/000920 also discloses that the composition is an anti-microbial composition for use in treating an infection. The hydrophilic clay may comprise one or more of smectite, laponite and bentonite clays, and preferably laponite.

### SUMMARY OF THE DISCLOSURE

The present invention is defined in the claims.

The present disclosure relates to the field of carrier and delivery systems for active molecular compounds. In particular, the present disclosure provides suspension formulations of aqueous dispersions for delivery to subjects (e.g., by injection).

The compositions of the present disclosure are non-toxic, stable, and suitable for in vivo use (e.g., oral use or injectable use). The compositions find use in the treatment of disease and in the delivery of active agents (e.g., drugs) via a variety of routes.

For example, in some embodiments, provide herein is a composition, comprising a suspension of a dispersion in

a suspender (e.g., glycerin), said dispersion comprising a dispersed phase within an aqueous phase, and wherein the dispersion comprises: a) a hydrophilic clay in the dispersed phase; and b) a quaternary ammonium compound in the dispersed phase. In some embodiments, the suspension comprises 0.05% (e.g., 0.01 to 0.1%) of said dispersion in a 1% (e.g., 0.5 to 5.0%) suspender (e.g., glycerin) solution in water. In some embodiments, the suspension is filtered (e.g., with a filter of 0.45 micron or smaller). In some embodiments, the suspension comprises or consists of particles of less than 0.45 micron in diameter. In some embodiments, the composition is non-toxic. In some embodiments, the composition is suitable for injection or oral use. In some embodiments, the hydrophilic clay comprises one or more of smectite, laponite and bentonite clays. In some embodiments, the quaternary ammonium compound comprises benzethonium chloride or derivatives thereof. In some embodiments, the dispersion further comprises an active agent (e.g., including but not limited to, one or more of: an antimicrobial, an analgesic, an anti-fungal, or an anti-inflammatory). In some embodiments, the dispersion and/or suspension comprises one or more of a chelating agent, emulsifier, humectants, moisturizer, detackifier, emollient, or thickener. In some embodiments, the dispersion is active (e.g., biologically active) in the absence of further active agents.

The present disclosure is not limited to particular manufacturing methods of the described dispersions and suspensions. In some embodiments, the dispersion is made by the method of i) dry mixing the hydrophilic clay and the quaternary ammonium compound to produce a mixture; ii) adding water to the mixture; and iii) incubating the mixture to form the dispersion. In some embodiments, the suspension is made by the method of a) mixing the dispersion with glycerin; b) adding water to make a solution; and c) filtering the solution.

The compositions described herein can be formulated for any number of uses or delivery methods. In some embodiments, the composition is a oral spray, an oral rinse, an ophthalmic drop, or a disinfectant, although other formulations are specifically contemplated.

Certain embodiments provide a method of making a suspension described herein, comprising the steps of: a) mixing a dispersion made by the method of i) dry mixing hydrophilic clay and a quaternary ammonium compound to produce a mixture; ii) adding water to the mixture; and iii) incubating the mixture to form the dispersion with glycerin; b) adding water to make a suspension; and c) filtering the suspension. In some embodiments, the suspension comprises 0.05% (e.g., 0.01 to 0.1%) of said dispersion in a 1% (e.g., 0.5 to 5.0%) glycerin in water solution.

Further embodiments provide a pharmaceutical composition comprising: a) a composition as described herein; and b) a pharmaceutically acceptable carrier.

Additional embodiments provide a kit, comprising: a) a composition as described herein; and b) an injection device. In some embodiments, the kit is provided in dosage form.

In some embodiments, provide herein is a bandage or dressing comprising a composition as described herein.

Yet other embodiments provide a method of administering an active agent to a subject, comprising: administering a composition as described herein into a subject in need thereof. In some embodiments, the composition is administered orally. In some embodiments, the composition is administered intravenously, subcutaneously, intramuscularly, etc.

Other embodiments provide a method of disinfecting a body part or surface, comprising: contacting a composition as described herein with said the part or surface (e.g., including but not limited to, a joint, an eye, a throat, a wound, a burn, or a surface of a medical device (e.g., a surgical instrument, an implantable medical device, a syringe, or a medication container).

Also provided is a method of treating an infection in a subject, comprising: administering a composition as described herein to the subject. In some embodiments, the infection is a blood infection (e.g., sepsis) and the composition is administered intravenously. In some embodiments, the infection is an injection by a bacteria, virus, or fungus. In some embodiments, the composition adsorbs endotoxins.

In further embodiments, provided herein is the use of a composition as described herein to deliver an active agent to a subject or to disinfect a body part or surface or treat an infection.

The present disclosure is not limited to particular subjects. In some embodiments, the subject is a human or a non-human animal.

Further embodiments are described herein.

## BRIEF DESCRIPTION OF THE DRAWINGS

FIG. 1 shows the bacterial adsorbence capacity of E. coli 8739 of compositions of embodiments of the present disclosure.

FIG. 2 shows the bacterial adsorbence capacity of E. coli of compositions of embodiments of the present disclosure.

FIG. 3 shows the $Log_{10}$ reduction at 96,000EU of compositions of embodiments of the present disclosure.

FIG. 4 shows the bacterial adsorbence capacity at 96,000EU of compositions of embodiments of the present disclosure.

FIG. 5 shows bactericidal activity of compositions of embodiments of the present disclosure against E. coli.
FIG. 6 shows bactericidal activity of compositions of embodiments of the present disclosure against S. marcescens.

## DEFINITIONS

As used herein, the term "dispersion" refers to a stable suspension. Such dispersions may be stable due to particle size and/or the presence of components having both hydrophilic and hydrophobic sites, e.g., as in a surfactant or emulsifier. In some embodiments, the dispersion is stable for more than one day, one week, one month, etc.

As used herein, the terms "continuous phase" and "dispersed phase" are related to a dispersion system, in which a first material is dispersed within a second material fine solid or liquid particles. In such a dispersion system, the term "continuous phase" refers to a first phase surrounding a second "dispersed phase." The "dispersed phase" refers to the suspended particles or liquid droplets dispersed in the continuous phase.

As used herein, the term "emulsion" refers to a heterogeneous system comprising a continuous phase and a non-continuous phase capable of forming droplets in the continuous phase.

As used herein, the term "emulsifier" refers to an agent that can reduce and/or eliminate the surface and the interfacial tension in a two-phase system. The emulsifier agent may possess both hydrophilic and lipophilic groups. The emulsifier may be considered to be either in the continuous phase, dispersed phase, or both.

As used herein, the phrase "in association with" is intended to include any or all of: chemical combination, charge attraction, entrapment, whole or partial dissolution, and suspension.

As used herein, the term "topically" refers to application of the compositions of the present disclosure to the surface of the skin, a wound, and/or mucosal cells or tissues (e.g., alveolar, buccal, lingual, masticatory, or nasal mucosa, etc.).

As used herein, the term "humectant" refers to a hygroscopic compound having a plurality of hydrophilic groups (e.g., glycerin (glycerol), propylene glycol, etc.).

As used herein, the term "moisturizer" refers to a composition that, when applied to a surface (e.g. skin, mucosal tissue, wound, etc.) causes retention of water within the surface.

As used herein, the term "subject" refers to any animal (*e.g.*, a mammal), including, but not limited to, humans, non-human primates, companion animals (e.g., dogs, cats, etc.), livestock (e.g., horses, cows, goats, sheep, pigs, etc.), rodents, birds, and the like, which is to be the recipient of a particular treatment.

As used herein, the term "pharmaceutical composition" refers to the combination of an active agent with a carrier, inert or active, making the composition especially suitable for diagnostic or therapeutic use *in vivo*, or *ex vivo.*

As used herein, the term "toxic" refers to any detrimental or harmful effects on a cell or tissue as compared to the same cell or tissue prior to the administration of the toxicant.

## DETAILED DESCRIPTION OF THE DISCLOSURE

The present disclosure relates to the field of carrier and delivery systems for active molecular compounds. In particular, the present disclosure provides suspension formulations of aqueous dispersions for delivery to subjects (e.g., by injection).

For example, in some embodiments, provided herein are suspension formulations of a dispersion. Exemplary dispersion formulations are described below and in US. Pat. No. 8,858 970.

Suspensions are prepared, for example, by taking mixing a dispersion described herein with a suspender (e.g., glycerin) in water solution and optionally filtering the solution. In some embodiments, the ratio of dispersion: suspender (e.g., glycerin): water is optimized in order to obtain an even suspension. During experiments described herein it was determined that 0.05% (e.g., 0.01 to 0.10%) dispersion in a 1% (e.g., 0.05 to 5%) glycerin suspension gave optimal performance. However, other concentration or ratios can be utilized.

It was further determined that filtering the suspension through a filter (e.g., 0.45 micron filter or other filter size) gave an even suspension that was non-toxic.

Accordingly, in some embodiments, the described suspensions are non-toxic, even suspensions that are suitable for injection and oral use. The suspensions find use in a variety of medical and industrial settings described herein.

In some embodiments, the present disclosure provides a suspension comprising a dispersion comprising a dispersed phase within a continuous or aqueous phase. In some embodiments, a continuous phase comprises a liquid or gel, and may optionally comprise one or more of a humectant, emollient, detackifier, moisturizer, thickener, chelating agent, or other additive. In some embodiments, a dispersed phase comprises: a hydrophilic particle substrate having electrically charged binding sites, an intermediate component comprising a hydrophobic moiety and an ionic moiety (e.g. electrically charged moiety, hydrophilic moiety), and optionally an active agent (e.g., biologically active agent (e.g., antimicrobial agent, etc.) comprising a hydrophobic moiety. In some embodiments, the ionic moiety of the intermediate component is attracted to the electrically charged binding sites of the hydrophilic particle substrate. In some embodiments,

the hydrophobic moiety of the intermediate component is attracted to the hydrophobic moiety of the active agent. In some embodiments, attractive forces between the intermediate component and both the hydrophilic particle substrate and active agent cause the components to assemble into a complex or supramolecular particle.

Exemplary dispersions are described below.

**Dispersions**

In some embodiments, compositions (e.g. dispersions) have viscosities of at least 100 centipoise (e.g., >100 centipoise, 100-150 centipoise, 150-200 centipoise, >200 centipoise, 200-300 centipoise, >300 centipoise, >500 centipoise). In some embodiments, compositions have viscosities of 150-200 centipoise. In some embodiments, compositions have viscosities of greater than about 1000 centipoise (e.g., 1000 centipoise... 2000 centipoise... 5000 centipoise... 10,000 centipoise... 20,000 centipoise... 50,000 centipoise... 100,000 centipoise... 200,000 centipoise, etc.). In some embodiments, compositions are gels. In some embodiments, desired viscosities are obtained by inclusion of gelling agents, high viscosity additives, and increase concentration of solids (e.g., >10%, >15%, >20%, >25%, >30%, >40%, >50%, etc.).

In some embodiments, a dispersion has a continuous aqueous phase containing particulate material held in suspension by small particle size, e.g., sub-micron, or by an emulsifier. In some embodiments, a dispersion has a suspended phase including suspended microparticles or smaller and may include hydrophobic droplets. The droplets and sometimes solid particles, may be held in suspension with the assistance of an emulsifier. In some embodiments, "continuous aqueous phase" or "aqueous phase" refers to the continuous phase surrounding solid particle and/or hydrophobic droplets. The aqueous phase thus contains suspended or dissolved components, e.g., thickeners, gelling agents, humectants, moisturizers, emulsifiers, chelating agents, stabilizers, adherents, emollients, dyes and fragrances. Emollients and emulsifiers may be considered as being in an intermediate phases between aqueous (polar) and non-polar (oil) phases. The particles and hydrophobic droplets may be considered to be the suspended phase. The water used to form the aqueous phase is deionized water obtained by any known means, e.g., ion exchange resins or distillation in an inert system, e.g., in non-reactive glass, or reverse osmosis.

In some embodiments, the compounds used for the intermediate components comprise ligands, i.e., bound to the central particle and arranged to accept additional components at exposed portions. In some embodiments, compound for use in an intermediate layer is a quaternary ammonium compound having a hydrophobic tail; although, any other compound having an ionic structure may be used and attracted as above described.

Ligands having antimicrobial properties include compounds having reactive inorganic cations, particularly those which have one or more electrons available for chemical reactions (e.g., transition metals) and compounds containing organic cations known to have bactericidal activity. For example, the antimicrobial effects of quaternary ammonium compounds, iodophor compounds, phenolics, alcohol, chlorine, peroxides, aldehydes and metals have been well documented. For further detail, see U.S. Patent No. 6,288,076.

Ligands having antimicrobial properties which are particularly desirable for use as ligands in the present disclosure include quaternary ammonium compounds, transition metals, organo metallic compounds, perchlorates, charged halogen-containing compounds, charged organic peroxides, ionic polymers, ionic surfactants, and mixtures thereof.

Especially desirable quaternary ammonium compounds include hexadecyltrimethyl ammonium bromide, trimethylphenyl ammonium chloride, and mixtures thereof. Especially desirable transition metals include copper, iron, manganese, zinc, silver, mercury, and mixtures thereof. The antimicrobial agent of the present disclosure includes ligands attached to the colloidal particles in excess of and up to 200% of the C.E.C. of the colloid particles (e.g., greater than 250% or greater than 300%), resulting in greater efficacy of the antimicrobial agent.

A preferred quaternary compound having cationic activity is benzethonium chloride. Benzethonium chloride, having an ionic hydrophilic site and a hydrophobic tail and being an antimicrobial and thus active may be used in both the secondary and tertiary layers. The benzethonium chloride may be present in an amount of, for example, 0.5% by weight of solids.

Quaternary ammonium compound may be used as a secondary layer and/or an antimicrobial tertiary layer, e.g., in an amount of, for example, 0.50 % by weight of solids.

When compounds have such properties, they may be directly and indirectly loaded onto the particle substrate in two layers. Complete loading on a single layer may be considered as a 100 % loading and when complete loading occurs on both the intermediate secondary and tertiary layers, loading may be considered to be 200%. The present disclosure may permit loading as high as 200% or even higher due to additional complex interactions.

In some embodiments, the present disclosure provides particle substrates. In some embodiments, particle substrates comprise hydrophilic particle substrates. In some embodiments, particle substrates comprise hydrophilic submicorn particle substrates. In some embodiments, particle substrates comprise electrically charged binding sites. In some embodiments, particle substrates comprise hydrophilic sites (e.g., hydrophilic binding sites). In some embodiments,

hydrophilic sites are due to ionic moieties, e.g., a quaternary, carboxy, sulfo, phosphor, or a polar component such as may be found in a chemically bound oxygen, nitrogen, sulfur or phosphorous atom having an exposed electron pair. Such components may be compounds or aggregations. Particular examples are anionic, cationic and non-ionic groups as may be found in surfactants. In some embodiments, particle substrates have submicron diameters (e.g., <1 μm, <0.5 μm, <0.2 μm, <0.1 μm, <0.05 μm, <0.02 μm, <0.01 μm, etc.). In some embodiments, submicron size provides stability. In some embodiments, submicron particles are nanoparticles that require no stabilization.

As used here, the term "particle substrate" means a particle that acts as a substrate for an interaction with an agent, compound, ligand, reactive group, functional group, etc. An example of particle substrates that find use in the present disclosure is a hydrophilic hydrated clay. Such clays are primarily aluminosilicates in which some of the aluminium and silicon ions have been replaced by elements with different valence, or charge. For example, aluminium ($Al^{3+}$) may be replaced by iron ($Fe^{2+}$) or magnesium ($Mg^{2+}$), leading to a net negative charge. This charge attracts positive cations which in turn may attract a corresponding anion.

The particles may be organic and inorganic particles, including nano-particles. Preferred inorganic materials have surface areas ranging from 50-1000 $m^2$/gm, with surface areas of 500-800 $m^2$/gm being especially desirable. Useful synthetic types of clay-type minerals include a synthetic hectorite, which is a layered hydrous magnesium silicate, such as LAPONITE (Southern Clay Products, Gonzales, Tex.), a synthetic mica-montmorillonite, such as BARASYM, (Baroid Division, NL Industries, Houston, Tex.) and mixtures thereof. Useful naturally occurring clay minerals include swelling clays such as aliettite, beidellite, bentonite, nontronite, saponite, sauconite, stevensite, swinefordite, volkonskoite, yakhontovite, hectorite, montmorillonite (such as BP colloid), and mixtures thereof. Other useful materials (both synthetic and naturally occurring) include, but are not limited to polymers, zeolites, layered double hydroxides, illite, chlorite, kaolinite, hydrotalcite, talc, halloysite, sepiolite, and palygorskite, as well as other minerals such as oxides, hydroxides, and silicates, to name just a few. Typically, the colloid particles of the present disclosure have a mean diameter of 1 nm to 100 microns, having mean diameters of less than 2 microns with diameters of less than one micron being preferred.

Preferred clays are hydrophilic smectite, laponite and bentonite clays having high cationic exchange properties. Other suitable particles are ion exchange resin particles, and organic plastic particles having charged sites.

In some embodiments, particles are characterized by both large surface areas and substantial ion exchange capacities. Such ion exchange capacities are usually, but not always cation exchange capacities (CEC). It is to be understood that anion exchange resins may also be used, e.g., polyfunctional resins containing quaternary amine groups. In general where "CEC" is used herein, it should be understood that anion exchange resins may also be used in the appropriate context. The number of binding sites on a particle may be determined by binding sites per mole when the structural formula of the resin is known as modified by surface characteristics, e.g., surface area due to particle sizes effects. A number of CEC's are known for particular materials, e.g., for laponites used in examples herein are known to have a CEC of about 55.0 meq/100 grams. Compositions provided herein are unique in that loadings well in excess of the CEC may be obtained, e.g., over 125 % up to as much as 250% or more.

Bioactive compositions, made according to the methods of the present disclosure use a variety of substrates, examples of which are given below, in addition to a variety of bioactive compounds that are attached to the substrate. By varying the organics that are used for ion exchange to prepare the organo-substrate, the organo-substrate can be tailored to have either hydrophilic or hydrophobic surface tension properties. Furthermore, by choosing the appropriate carrier substrate, e.g., clay, that is used for additional attachment of organic onto the organo-substrate, the antimicrobials produced can exhibit either hydrophilic or hydrophobic properties. This allows the compositions to be used in either aqueous or non-aqueous formulations.

In certain embodiments, active agents comprise a tertiary layer of particulates of the present disclosure. In some embodiments, active agents (e.g., molecules for forming the tertiary layer) have a hydrophobic moiety (e.g., hydrophobic tail). In other embodiments, active agents (e.g., molecules for forming the tertiary layer) have a biologically active moiety. In particular embodiments, active agents are quaternary compounds. In some embodiments, active agents are antimicrobials, humectants, moisturizers, anti-inflammatory, and nutrients.

In some embodiments, a quaternary compound comprises one or more antimicrobial agents, including, but not limited to: lauryl dimethyl benzylammonium chloride, benzalkonium chloride, alkyltrimethyl ammonium chloride, dialkyldimethylammonium chloride, alkyldimethylbenzylammonium chloride, alkyldimethyl(ethylbenzyl)ammonium chloride, combinations thereof, etc. In other embodiments, a quaternary compound comprises one or more non-antimicrobial conditioning agents, including, but not limited to: cetrimide, cetrimonium bromide, cetylamidopropyldimethyl ammonium chloride, stearyl trimethyl ammonium chloride, stearalkonium chloride, dihydrogenated tallow dimethyl ammonium chloride, combinations thereof, etc.

Specific, non-limiting examples of suitable hydrophobic active ingredients are: acetretin, albendazole, albuterol, aminoglutethimide, amiodarone, amlodipine, amphetamine, amphotericin B, atorvastatin, atovaquone, azithromycin, baclofen, beclomethasone, benezepril, benzonatate, betamethasone, bicalutanide, budesonide, bupropion, busulfan, butenafine, calcifediol, calcipotriene, calcitriol, camptothecin, candesartan, capsaicin, carbamezepine, carotenes, celecoxib, cerivastatin, cetirizine, chlorpheniramine, cholecalciferol, cilostazol, cimetidine, cinnarizine, ciprofloxacin, cisapride, clarithromycin, clemastine, clomiphene, clomipramine, clopidogrel, codeine, coenzyme Q10, cyclobenzaprine, cyclosporin, danazol, dantrolene, dexchlorpheniramine, diclofenac, dicoumarol, digoxin, dehydroepiandrosterone, dihydroergotamine, dihydrotachysterol, dirithromycin, donezepil, efavirenz, eposartan, ergocalciferol, ergotamine, essential fatty acid sources, etodolac, etoposide, famotidine, fenofibrate, fentanyl, fexofenadine, finasteride, fluconazole, flurbiprofen, fluvastatin, fosphenytoin, frovatriptan, furazolidone, gabapentin, gemfibrozil, glibenclamide, glipizide, glyburide, glimepiride, griseofulvin, halofantrine, ibuprofen, irbesartan, irinotecan, isosorbide dinitrate, isotretinoin, itraconazole, ivermectin, ketoconazole, ketorolac, lamotrigine, lansoprazole, leflunomide, lisinopril, loperamide, loratadine, lovastatin, L-thryroxine, lutein, lycopene, medroxyprogesterone, mifepristone, metfloquine, megestrol acetate, methadone, methoxsalen, metronidazole, miconazole, midazolam, miglitol, minoxidil, mitoxantrone, montelukast, nabumetone, nalbuphine, naratriptan, nelfinavir, nifedipine, nilsolidipine, nilutanide, nitrofurantoin, nizatidine, omeprazole, oprevelkin, oestradiol, oxaprozin, paclitaxel, paracalcitol, paroxetine, pentazocine, pioglitazone, pizofetin, pravastatin, prednisolone, probucol, progesterone, pseudoephedrine, pyridostigmine, rabeprazole, raloxifene, rofecoxib, repaglinide, rifabutine, rifapentine, rimexolone, ritanovir, rizatriptan, rosiglitazone, saquinavir, sertraline, sibutramine, sildenafil citrate, simvastatin, sirolimus, spironolactone, sumatriptan, tacrine, tacrolimus, tamoxifen, tamsulosin, targretin, tazarotene, telmisartan, teniposide, terbinafine, terazosin, tetrahydrocannabinol, tiagabine, ticlopidine, tirofibran, tizanidine, topiramate, topotecan, toremifene, tramadol, tretinoin, troglitazone, trovafloxacin, ubidecarenone, valsartan, venlafaxine, verteporfin, vigabatrin, vitamin A, vitamin D, vitamin E, vitamin K, zafirlukast, zileuton, zolmitriptan, zolpidem, and zopiclone. In addition, salts, isomers and derivatives of the above-listed hydrophobic active ingredients may also be used, as well as mixtures.

In some embodiments, the dispersion further comprises an active agent that is a drug (e.g., anti-inflammatory drug, analgesic, antibiotic, and the like).

Dispersions may comprise additional compounds including, but not limited to, emulsifiers, chelating agents, gelling agents, stabilizers, adherents, emollients, dyes, detackifiers, thickeners, nonaqueous moisturizers, anti-inflammatory agents, skin adherents, and fragrances.

Dispersions may also comprise a detackifier such as phenyl substituted silicone fluid, e.g., phenyl trimethicone. In some embodiments, a detackifier also acts as a humectant. In some embodiments, dispersions comprise an emollient, e.g., pentaerythrityl tetracaprylate.

In certain embodiments, a dispersion comprises one or more dyes and/or pigments, including, but not limited to: titanium dioxide, natural minded and synthetic iron oxides, blends of inorganic oxides and fillers (kaolin, talc, silica, mica), D&C colors, FD&C colors, combinations thereof, etc. In some embodiments, a dispersion comprises one or more dyes, including, but not limited to: Disperse Red 13, Disperse Green 9, Solvent Black 3, Disperse Blue 148, Disperse Violet 63, Disperse Blue, Disperse Blue 14, Solvent Orange 15, Solvent Orange 7, Solvent Blue 14, Disperse Yellow 82, 9-diethylamino-5H-benzo[alpha]phenoxazine-5-one, 1-dimethylamino-5-sulfamoyl-naphthalene, pyrene, 1-pyrenecarbaldehyde, Reichardt's dye, 4-aminophthalimide, 4-(N,N-dimethylamino)phthalimide, bromonapthalene, 2-(dimethylamino) naphthalene, solvatochromatic dye, combinations thereof, etc.

In particular embodiments, a dispersion comprises one or more fragrances, including, but not limited to: tea tree oil, citrus oils (e.g., lemon oil, orange oil, etc.), oils from herbs (e.g., rosemary, oil, thyme oil, oregano oil, etc.), oils from woods (e.g., rosewood oil, cedarwood oil), cinnamaldehydes or cinnamon bark oil, eugenol or clove flower oil, cineol or eucalyptus oil, camphor or camphor tree oil, geraniol or palmarosa oil, citronella oil, geranium oil, cedrol, etc. In some embodiments, the present disclosure provides any suitable essential oil. In some embodiments, fragrances further provide antimicrobial, fungicidal, and/or insect-repelling functionality.

In some embodiments, a dispersion comprises one or more emulsifiers, including, but not limited to: PEG-dimethicones, polyglycerol dimethicones, Sorbian oleate, glyceryl esters, C12-15 alkyl benzoate, castor oil, cetearyl alcohol, cetyl alcohol, cetyl esters, cetyl palmitate, diisopropyl adipate, emu oil, isopropyl myristate, isopropyl palmitate, lanolin, mangifera indica seed butter, mineral oil, myristyl myristate, sorbitan oleate, safflower oil, shea butter, stearic acid, stearyl alcohol, calcium stearoyl lactylate, ceteareth-20, cocamide MEA, glyceryl laurate, glyceryl stearate, glyceryl stearate and PEG-100 stearate, glyceryl stearate SE, glycol distearate, glycol stearate, isoceteth-20, isosteareth-20, lauramide DEA, laureth-23, laureth-4, linoleamide DEA, methyl glucose sesquistearate, oleth-10, oleth-10/polyoxyl 10 oleyl ether NF, Oleth-2, Oleth-20, PEG-100 Stearate, PEG-20 Almond Glycerides, PEG-20 Methyl Glucose Sesquistearate, PEG-25 Hydrogenated castor oil, PEG-30 dipolyhydroxystearate, PEG-4 dilaurate, PEG-40 sorbitan peroleate, PEG-60 almond glycerides, PEG-laurate, PEG-80 sorbitan laurate, polysorbate 20, polysorbate 60, polysorbate 80, polysorbate 85, sodium stearoyl lactylate, sorbitan isostearate, sorbitan laurate, sorbitan sesquioleate, sorbitan stearate, sorbitan stearate and sucrose cocoate, sorbitan trioleate, stearamide MEA, steareth-2, steareth-21, combinations thereof, etc.

Dispersion may comprise one or more humectants, including, but not limited to: polyglycerol dimethicones, gelatin, glycerin, honey, hyaluronic acid, panthenol, propylene glycol, sodium ammonium lactate, sodium pyrrolidine carboxylic acid, sorbitol, urea, 1,2,6 hexanetriol, Hexylene and Butylene Glycol, Dipropylene glycol, Hexylene Glycol, Panthenol, Phytantriol, Sodium PCA, Triethylene glycol, olyglyceryl sorbitol, Glucose, Fructose, Polydextrose, Potassium

PCA, Hydrogenated Honey, Inositol, Hexanediol beeswax, Hexanetriol Beeswax, Hydrolyzed Elastin, Hydrolyzed Collagen, Hydrolyzed Silk, Hydrolyzed Keratin, Erythritol, Capryl glycol, Isoceteth-(3-10, 20, 30), Isolaureth-(3-10, 20, 30), Laneth-(5-50), Laureth-(1-30), Steareth-(4-20), Trideceth-(5-50), sucrose, glucose, aloe, alpha-hydroxy acids (AHA's), combinations thereof, etc.

In some embodiments, a dispersion comprises one or more thickeners and/or stabilizers, including, but not limited to: dimethicone gums, dimethicone cross-polymers, stearic acid, stearic acid with cetyl alcohol, cellulose, carbopol, polyacrylic acid, clays, carrageenan, pectin, and locust bean gum, xanthum gum, carbomer (a homopolymer of acrylic acid with a high molecular weight, which is cross-linked with any of several polyalcohol allyl ethers), combinations thereof, etc.

In certain embodiments, a dispersion comprises one or more detackifiers and/or emollients, including, but not limited to: dimethicone cross-polymers, cyclomethicone, plant oils, polyisobutene, squaline, ceramides like lacto-ceramide, essential fatty acids (linoleic acid), fatty acids and esters of fatty alcohols and fatty acids, lanolin, lauric acids, stearic and palmitic acids with carbon chains lengths of 16 and 18 (coconut oil, grapeseed oil, and palm kernel oil), ceramides, combinations thereof, etc. In some embodiments proteins are providedthat, like emoillents, shrink on the skin (or a wound) leaving a film that smoothes the skin, thereby avoiding water loss (e.g., collagen, keratin, elastin, protein mixtures like wheat protein).

In various embodiments, a dispersion comprises one or more alcohols, including, but not limited to: acyclic alcohols (e.g., ethanol), isopropyl alcohol, etc.

In other embodiments, a dispersion comprises one or more adherents and/or film formers, including, but not limited to: trimethylsiloxysilicates, acrylates/dimethicones, etc.

In some embodiments, a dispersion comprises one or more conditioners, including, but not limited to: dimethicone gums, amine modified silicones, cetrimide, cetrimonium bromide, cetylamidopropyldimethyl ammonium chloride, combinations thereof, etc.

In particulat embodiments, a dispersion comprises one or more preservatives, including, but not limited to: Phenonip, Parabens and esterof parabenzoic acids (phenoxyethanol), antioxidants (tocopherol, BHT, combinations thereof, etc.

In various embodiments, a dispersion comprises one or more oils and/or waxes, including, but not limited to: aleurites moluccana seed oil, almond oil NF, anhydrous lanolin USP, apricot kernel oil, avocado oil, babassu oil, beeswax, borage seed oil, brazil nut oil, cannibas sativa seed oil, canola oil, caprylic/ capric triglyceride, carrot seed oil, ceresin, coconut oil, daucus carota sativa root extract, dimethicone, dog rose hips oil, evening primrose oil, grape seed oil, hybrid safflower oil, jojoba oil, macadamia nut oil, mangifera indica seed butter, olive oil, oryza sativa oil, peanut oil NF, petrolatum, PPG-15 steryl ether, retinyl palmitate, sesame oil, soybean oil, sunflower oil, sweet almond oil, theobroma cacao seed butter, tocopherol, combinations thereof, etc.

In some embodiments, the compositions may also contain a moisturizing agent (moisturizer). Moisturizers prevent and treat dry skin, protect sensitive skin, improve skin tone and texture, and mask imperfections. Moisturizers are often complex mixtures of chemical agents specially designed to make the external layers of the skin (epidermis) softer and more pliable, by increasing its water content by reducing evaporation. Naturally occurring skin lipids and sterols, as well as artificial or natural oils, humectants, emollients, and lubricants, for example may be part of the moisturizer composition. One example of a moisturizing substance that is used in the present disclosure is glycerin. Other skin moisturizers that are considered for use in the present disclosure include, but are not limited to: urea, ammonium lactate, NaPCA, saccharides, simple sugars, hydroscopic salts and glycerin, often found in combination with an emollient such as Butyrospernum Parkii (Shea butter) fruit, glycine soja (soybean) sterol and Helianthus Annuus (hybrid sunflower) oil and olive oil. As previously mentioned, another suitable moisturizer is gluconolactone combined with sodium benzoate.

Chelating agents, e.g., a gluconate, may be used to chelate substances that may interfere with desired reactions and combinations, e.g., The chelating agent may be present to attract compounds that may interfere with the binding of the quaternary compound with the colloidal substrate Chelating agents are not usually required. A chelating agent, e.g., 20% of a 60% solution of gluconic acid and sodium gluconate as determined after neutralization, may be present.

Some embodiments provide dispersions in the form of a topical antimicrobial carrier system wherein the particulate material includes a hydrophilic clay, the secondary layer comprises a quaternary ammonium compound and the tertiary layer comprises an antimicrobial compound. In some embodiments, dispersions comprise a quaternary compound such as benzethonium chloride. In some embodiments, dispersions are in the form of a topical anti-inflammatory composition, e.g., where the tertiary layer contains an anti-inflammatory compound such as an omega 3 fatty acid. In some embodiments, dispersions are in the form of a topical analgesic, e.g., where the tertiary layer contains capsaicin or lidocaine.

In some embodiments, dispersions or suspensions for use in injection comprise pharmaceutically acceptable carriers. For example, compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

In some embodiments, dispersions or suspensions for use in oral care products comprise one or more

components commonly found in oral care products (e.g., mouthwash, toothpaste, etc.). Examples include, but are not limited to, cetylpyridinium chloride, chlorhexidine, essential oils, fluoride, peroxide, alcohol, and flavorings.

In some embodiments, dispersions or suspensions for use in ophthalmic products contain one or more components commonly found in ophthalmic products. Examples include, but are not limited to, saline, steroids, antihistamines, sympathomimetics, beta receptor blockers, parasympathomimetics, parasympatholytics, prostaglandins, nonsteroidal anti-inflammatory drugs (NSAIDs), antibiotics, antifungal, or topical anesthetics.

Dispersions of the present disclosure provide significant advantages over administration of active agents via other carriers and/or systems, including, but not limited to: increased adsorption capacity for bacterial cells and toxins, reduction of toxicity of active ingredients, extended time of activity for active ingredients, time release characteristics, controlled release of active ingredients, ease of use, increased active ingredient loads of up to 200% of ion exchange capacity or greater, reduced irritation and enhanced effectiveness.

The methods of the present disclosure may include the steps of combining a hydrophilic clay in the form of particles having charged sites, with a compound having an ionic moiety and a hydrophobic tail to form a pre-combination and introducing the pre-combination into an aqueous phase to obtain an intermediate dispersion and combining a hydrophobic active compound with the intermediate dispersion to obtain further dispersion of particles having a substrate particle combined with an intermediate secondary layer having a hydrophobic tail and a tertiary layer including the active compound.

Further examples of detailed methods of making and using a dispersion of the disclosure include, but are not limited to the following. In certain embodiments the present disclosure provides methods of making a dispersion including one or more of the steps of: adding a humectant to deionized water to form an aqueous suspension; uniformly mixing a hydrophilic clay and a quaternary compound; adding water to the resulting clay and quaternary compound mixture to form a hydrophilic clay-quaternary ammonium compound combination; combining the aqueous suspension and the hydrophilic clay-quaternary ammonium compound combination to obtain a suspension; heating the suspension to between 70 and 90° C; optionally dispersing together at least one active agent to obtain a dispersion; heating the dispersion to between 70 and 90° C; mixing the dispersion with the suspension; drawing a vacuum; and homogenizing the resulting composition. In some embodiments the present disclosure provides methods of making a carrier system for a biologically active compound having a cationic moiety and a hydrophobic moiety including one or more of the steps of: mixing the biologically active compound and a hydrophilic clay to form an active compound-hydrophilic clay mixture; suspending the active compound-hydrophilic clay mixture in an aqueous liquid to form a suspension; and incorporating the suspension into a carrier. Any of the above or following methods may include adjusting pH if necessary, e.g., by adding KOH to mixed contents to adjust the pH to from about 5.2 to 6.2.

**Methods**

The present disclosure finds use in a variety of applications and compositions. Exemplary applications and compositions are provided below. These should not be viewed as limiting; rather, the alterations and combinations of these embodiments are within the scope of the disclosure.

The suspensions described herein find use in a variety of applications. Examples include, but are not limited to, injectable applications, ingestible applications, oral care applications, ophthalmic applications, wound applications, surgical applications, burn applications, and industrial applications.

For example, in some embodiments, the suspensions find use in delivering an active agent (e.g., drug) via injection or topically, to the eye, throat, wound, or burn.

In some embodiments, the suspension serves as an anti-microbial agent without the addition of further active agents. Such suspensions find use, for example, in killing bacteria in the throat, eye, wounds, or inside the body (e.g., sepsis). In some embodiments, the suspension adsorbs endotoxins (e.g., in the blood).

In some embodiments, the suspensions find use in disinfecting a medical device (e.g., including but not limited to, a surgical instrument, an implantable medical device, a syringe, or a medication container (e.g., IV bag)).

Additional applications of the suspension formulations described herein are provided below.

The disclosure also includes a method for topically disinfecting an epidermal surface including the step of contacting the epidermal surface with a composition of the disclosure where a tertiary layer contains an antimicrobial compound.

In a further embodiment, the disclosure includes a method of topically applying a medicament to an epidermal surface comprising applying a composition of the disclosure wherein a tertiary layer contains the medicament.

In some embodiments, the compositions are provided as antimicrobial compositions. The compositions find use with subjects, including, but not limited to: healthcare workers, nurses, doctors, veterinarians, surgeons, patients, pre- and postoperative patients, schools, teachers, students, travelers, flight attendants, sanitation workers etc. Antimicrobial compositions (e.g. comprising suspensions described herein) may find use in protecting against the spread of and/or killing a wide variety of pathogens including, but not limited to: gram positive bacteria (e.g., *Staphylococcus aureus,*

*Methicillin-resistant Staphylococcus aureus, Staphylococcus epidermidis, Streptococcus faecalis, Clostridium spp., Listeria spp., Bacillus spp.,* etc.), gram negative bacteria (e.g., *E. coli, Serratia marcescens, Pseudomonas aeruginosa, Salmonella spp., Helicobacter, Acinetobacter Baumanii,* etc.), yeast (e.g., *Candida albicans, Cryptococcus Neoformans, Candida glabrata,* etc.), mold (e.g., *Aspergillus niger, Cladosporium, Acremonium,* etc.), viruses (e.g., Adenovirus, Influenza virus, Rotavirus, Rhinovirus, etc.), etc. In some preferred embodiments, the active agent in antimicrobial compositions is benzethonium chloride.

The compositions described herein are provided for any number of delivery systems. In some embodiments, compositions are formulated for injection (e.g., intravenous, intramuscular, epidural, intra-arterial, intraperitoneal, intrathecal, or subcutaneous administration). In some embodiments, compositions for injection are formulated as pharmaceutical compositions as described above.

In some embodiments, such are provided in the form of a kit comprising the suspension, including one or more active agents, and an administration component or device. In some embodiments, compositions suitable for injection are provided with a syringe, autoinjector, iv delivery device, infusion device, and the like. In some embodiments, compositions for topical delivery are provided with a device for spraying or drinking the composition. In some embodiments, kits comprise a unit dose form of a composition.

In some embodiments, antimicrobial compositions find use in the treatment of infectious disease (e.g., by a virus, fungus, or bacteria) in vivo (e.g., blood borne or other in vivo infections).

In some embodiments, compositions described herein find use in the treatment of veterinary infections. Veterinary compositions are administered via, for example, feed additives, liquid, injection, oral, drenching, collars, dips, dust bags, etc.

The compositions described herein find use in the treatment of any number of infections in primates, livestock, companion animals, etc. Examples of common livestock diseases and infectious agents include, but are not limited to, bovine respiratory disease complex (BRDC), Clostridial disease, BRSV (bovine respiratory syncytial virus), BVD (bovine viral diarrhea), *Haemophilus somnus,* IBR (infectious bovine rhinotracheitis), PI3 (Parainfluenza Type 3), *Pasteurella haemolytica* and *Pasteurella multocida,* Brucellosis and tuberculosis, foot and mouth disease, tetanus, equine encephalomyelitis virus, equine influenza, equine herpesvirus: EHV-1 and EVH-4, west nile virus, respiratory infections caused by *Streptococcus suis, Pasteurella,* and *Actinobacillus pleuropneumoniae, Brachyspira hyodsenteriae,* mastitis, and parvovirus.

Examples of common diseases and infectious agents in companion animals include, but are not limited to, parvovirus, rabies, ringworm, *Brucella canis,* canine distemper virus (CDV), Leptospirosis, Ehrlichiosis, feline immunodeficiency virus (FIV), and Feline Leukemia Virus (FelV).

It is to be understood that this disclosure is not limited to the particular methodology, materials and modifications described and as such may, of course, vary. It is also understood that the terminology used herein is for the purpose of describing particular aspects only, and is not intended to limit the scope of the present disclosure, which is limited only by the appended claims. Although any methods, devices or materials similar or equivalent to those described herein can be used in the practice or testing of the disclosure, they should not be viewed as limiting.

**EXPERIMENTAL**

The following Examples utilize a laponite/benzethonium chloride dispersion (BC200). BC200 was prepared by the following method:

1 6.3 g of Benzethonium Chloride (BTC) was added to a 1 L bottle.
2 463.0 g of deionized (DI) water was added to the bottle, then mixed to dissolve the BTC.
3 25.0 g of LAPONITE RDS was added to the bottle and mixed.
4 The bottle was placed into 60C oven for 24 hours.
5 The bottle was centrifuged for 5 minutes at 3500 rpms and the supernant decanted.
6 A second solution of BTC (6.3 g of BTC and 463.0 g of DI) was added and mixed.
7 The bottle was placed back into th 60C oven for 24 hours.
8 The bottle was centrifuged for 5 minutes at 3500 rpms and the supernant decanted.
9 A third solution of BTC (6.3 g of BTC and 463.0 g of DI) was added and mixed.
10 The bottle was placed back into th 60C oven for 24 hours.
11 The bottle was centrifuged for 5 minutes at 3500 rpms and the supernant decanted.
12 463.0 g of DI water was added to the bottle, then mixed. This is wash 1.
13 The bottle was centrifuged for 5 minutes at 3500 rpms and the supernant decanted.
14 Steps 12 and 13 were repeated twice more. This is wash 2 and 3.

**Example I**

This Example describes preparation of an injectable formulation of a dispersion. Manufacturing Process: 900g of laponite/benzethonium chloride dispersion (See e.g., US. Pat. No. 8,858 970 was weighed out. Sample 1 was 0.05% dispersion (BC200), 1.0% Glycerin, and 98.9% Sterile Water (0.011% available BZT). Sample 2 was 0.5% BC200, 10% Glycerin, and 89.0% Sterile Water (0.1100% available BZT). The sample was brought up to 10g with glycerin. One mL was transferred to a 100mL volumetric flask and QS'd with Water. The solution was filtered through a 0.45 micron filter via vacuum filtration.

HPLC Extraction Method: One mL of sample is transferred into a 20mL, volumetric flask, QS'd with methanol, and filtered twice through a 0.45 micron filter using a syringe. One mL of the final solution is used for HPLC analysis Theoretical Recovery Calculations for a dispersion (Busumite dispersion) 1.9 g of Busumite in Glycerin

$$\xrightarrow{\text{1 ml into 100mL H2O}}$$

0.19 g total or 0.0019 g/mL 11% of Basumite is Benzethonium Chioride 0.0019g/mL X 0.11 = 0.000209 g/mL X 1000 = 0.209 mg/mL of Benzethonium Chloride 1mL of this final solution is transferred into 20mL for extraction

$$\frac{0.209\text{mg/mL}}{20\text{ mL}} \quad \text{X } 1000 = 10.45 \text{ ug/mL}$$

HPLC Data

Sample 1

Unfiltered Recovery - 10.3257 ug/mL
Filtered Recovery - 5.2723 ug/mL

Sample 2

Unfiltered Recovery - 11.1991 ug/mL
Filtered Recovery - 5.0742 ug/mL

The data shows a complete recovery of both layers in the unfiltered samples. Recovery in the filtered samples show a ~50% loss during filtration. An even suspension of BC200 to glycerin in water was achieved by means of decreasing the particle size via an optimal ratios of BC200:Glycerin:Water and decreasing particles size via filtration (getting rid of any agglomerations).

Cytotoxicity tests were performed on the suspensions using USP guidelines. Samples were vortexed before testing. After the 48 - 72 hour plating period, the growth medium from duplicate 10 $cm^2$ wells, each containing a monolayer of L-929 mouse fibroblast cells (ATCC Cell Line CCL 1, NCTC Clone 929), was aspirated and replaced with 2 mL of Dulbecco's Phosphate Buffered Saline (PBS) to wash away any debris. The PBS was aspirated and replaced with 0.8 mL of 5% serum supplemented cell culture medium (MEM). Neutral Red stain was also mixed with the MEM prior to transfer to the cell culture wells. For every 2 mL of MEM, 20 $\mu$L of Neutral Red stain was added. A sterile filter paper with a flat surface measuring 1.0 cm2 total surface area was saturated with approximately 0.1 mL of the test article and placed directly on the cell culture monolayer in the center of a 10 $cm^2$ well. Duplicate preparations were prepared.

Negative Control Article Description and Preparation: USP Reference Standard High Density Polyethylene (HDPE) was used as a negative control. The negative control was autoclaved at Pacific BioLabs prior to use. The negative control (measuring 1.0 $cm^2$) was placed in the center of a 10 $cm^2$ well containing mouse fibroblast cells. The negative control was prepared in duplicate.

Positive Control Article Description and Preparation: The Latex Surgical Glove was used as a positive control. The positive control (measuring 1.0 cm2) was placed in the center of a 10 $cm^2$ well containing mouse fibroblast cells. The positive control was prepared in duplicate.

Incubation: All wells were incubated in a humidified incubator with 5 $\pm$ 1% $CO_2$ at 37 $\pm$ 1°C for not less than 24

hours.

Scoring: After incubation, the test articles and controls were removed from the wells. The cell cultures were examined under an inverted light microscope at 100X magnification for cytotoxic response.

The filtered dispersion of sample 1 exhibited no cytotoxicity. An unfiltered dispersion exhibited moderate cytotoxicity.

**Example 2**

This example describes anti-bacterial activity of BC200. Table 1 shows bacterial adherence of BC200 and benzethonium laponite. FIGS. 1-4 show bacterial adsorbance of BC200 and benzethonium laponite. Tables 2 and 3 show adsorption capacity and efficiency of a variety of materials. Table 2 shows experimental details and Table 3 shows results. Tables 4-5 and FIGS. 5-6 shows bactericidal activity of a variety of materials. FIG. Table 4 and FIG. 5 shows activity against *E. coli.* Table 5 and FIG. 6 show activity against *S. marcescens.*

**Table 1**

| Product | Test Product (mg) | n | Test Product Concentration (% w/v) | Starting Test Organism (CFU) | Test Organism Adherence, Adh (CFU) | Adherence Capacity, AdhC (CFU/mg) |
|---|---|---|---|---|---|---|
| BC100[a] | 8.0 | 2 | 0.1 | $7.24 \times 10^7$ | $1.38 \times 10^7$ | $1.71 \times 10^6$ |
| BC200[b] | 8.0 | 2 | 0.1 | $7.34 \times 10^7$ | $1.38 \times 10^8$ | $3.16 \times 10^6$ |

[a] BC100 is Benzethonium Laponite abbreviated.
[b] BC200 is Boemite™ abbreviated.

| Product | Test Product (mg) | n | Test Product Concentration (% w/v) | Starting Test Organism (CFU) | Test Organism Adherence, Adh (CFU) | Adherence Capacity, AdhC (CFU/mg) |
|---|---|---|---|---|---|---|
| BC100[a] | 8.0 | 2 | 0.1 | $7.24 \times 10^7$ | $1.38 \times 10^7$ | $1.71 \times 10^6$ |
| BC200[b] | 8.0 | 2 | 0.1 | $7.24 \times 10^7$ | $1.38 \times 10^8$ | $3.16 \times 10^6$ |

[a] BC100 is Benzethonium Laponite abbreviated.
[b] BC200 is Boemite™ abbreviated.

| Product | Test Product (mg) | n | Test Organism | Test Product Concentration (% w/v) | Starting Test Organism (CFU) | Test Organism Adherence, Adh (CFU) | Adherence Capacity, AdhC (CFU/mg) |
|---|---|---|---|---|---|---|---|
| BC200[a] | 8.0 | 2 | *Escherichia coli* 8739 | 0.1 | $7.24 \times 10^7$ | $1.38 \times 10^7$ | $1.71 \times 10^6$ |
| BC200[a] | 8.0 | 2 | *Salmonella typhimurium* | 0.1 | $3.40 \times 10^8$ | $2.14 \times 10^7$ | $2.6 \times 10^6$ |
| BC200[a] | 8.0 | 2 | *Serratia marcescens* | 0.1 | $7.90 \times 10^7$ | $3.40 \times 10^6$ | $4.23 \times 10^5$ |
| BC200[a] | 8.0 | 2 | *Pseudomonas aeruginos 9027* | 0.1 | $2.51 \times 10^8$ | $8.22 \times 10^7$ | $1.03 \times 10^7$ |
| BC200[a] | 8.0 | 2 | *Staphylococcus aureus* 6538 | 0.1 | $6.88 \times 10^7$ | $1.14 \times 10^7$ | $1.36 \times 10^6$ |
| BC200[a] | 8.0 | 2 | *Staphylococcus aureus* 33593 (MRSA) | 0.1 | $2.70 \times 10^8$ | $3.87 \times 10^7$ | $4.73 \times 10^6$ |

[a] BC200 is Boemite™ abbreviated.

**Table 2**
**Setup**

| Sorbent | n | Sorbent (mg) | LPS, Used (EU) ± SD* |
|---|---|---|---|
| PMB[a] | 6 | 38.50 | 96,528 ± 9,726 |
| BC200[b] | 6 | 38.03 | |
| BC100[c] | 6 | 39.02 | |
| AWC[d] | 6 | 39.51 | |

[a] Polymycin B, -sulfate

[b] Bioclay BZT/Busumite™, highly loaded organoclay

[c] Benzethonium Laponite, organoclay

[d] Acclaym Wound Care 0.5% available BZT

*STDEV

**Table 3**
**Results**

| Sorbent | LPS Conc.Post Incubation (EU/ml) ± SD* | $Log_{10}$ Reduction ± SD* | Adsorption Capacity (EU/mg) ± SD* | Adsorption Efficiency, $E$ (%) ± SD* |
|---|---|---|---|---|
| PMB[a] | 760 ± 265 | 0.538 ± 0.142 | 1,746 ± 265 | 69.66 ± 10.58 |
| BC200[b] | 7 ± 6 | 2.69 ± 0.38 | 2,531 ± 6 | 99.70 ± 0.20 |
| BC100[d] | 765 ± 321 | 0.54 ± 0.17 | 1,718 ± 319 | 69.40 ± 12.90 |
| AWC[e] | 159 ± 72 | 1.25 ± 0.24 | 2,288 ± 70 | 93.7 ± 2.9 |

[a] Polymycin B, -sulfate

[b] Bioclay BZT/Busumite™, highly loaded organoclay

[c] Benzethonium Laponite, organoclay

[d] Acclaym Wound Care made with BC200

*STDEV

**Table 4**

| Product | Test Product (mg) | n | Test Product Concentration (% w/v) | Starting Test Organism, (CFU) | Test Organism Kill, (CFU) | $Log_{10}$ Reduction | Percent Reduction, (%) |
|---|---|---|---|---|---|---|---|
| BC100[a] | 8.0 | 2 | 0.1 | $1.23 \times 10^8$ | $1.42 \times 10^7$ | 0.05 | 11.52597 |
| BC200[b] | 8.0 | 2 | 0.1 | $1.23 \times 10^8$ | $1.23 \times 10^8$ | 8.09 | 99.99995 |
| BC200[b] | 4.0 | 2 | 0.05 | $1.23 \times 10^8$ | $1.23 \times 10^8$ | 4.86 | 99.99860 |
| BC200[b] | 2.0 | 2 | 0.025 | $1.23 \times 10^8$ | $1.23 \times 10^8$ | 3.22 | 99.93994 |

[a] BC100 is Benzethonium Laponite abbreviated.

[b] BC200 is Busumite™ abbreviated.

**Table 5**

| Product | Test Product (mg) | n | Test Product Concentration (% w/v) | Starting Test Organism, (CFU) | Test Organism Kill, (CFU) | $Log_{10}$ Reduction | Percent Reduction, (%) |
|---|---|---|---|---|---|---|---|
| BC100[a] | 8.0 | 2 | 0.1 | $1.78 \times 10^8$ | $2.38 \times 10^8$ | 0.06 | 13.37079 |
| BC200[b] | 8.0 | 2 | 0.1 | $1.78 \times 10^8$ | $1.78 \times 10^8$ | 7.25 | 100.00000 |
| BC200[b] | 4.0 | 2 | 0.05 | $1.78 \times 10^8$ | $1.78 \times 10^8$ | 5.47 | 99.99966 |
| BC200[b] | 2.0 | 2 | 0.025 | $1.78 \times 10^8$ | $1.78 \times 10^8$ | 4.44 | 99.99640 |

[a] BC100 is Benzethonium Laponite abbreviated.

[b] BC200 is Busumite™ abbreviated.

## Claims

1. A composition, comprising:

   a suspension of a dispersion in glycerin, said dispersion comprising a dispersed phase within an aqueous phase, and wherein the dispersion comprises:

   a) a hydrophilic clay in said dispersed phase; and
   b) a quaternary ammonium compound in said dispersed phase,

   wherein said suspension comprises 0.01% to 0.1% of said dispersion in a 0.5% to 5% glycerin in water solution,
   wherein said suspension is filtered, wherein said filter is 0.45 micron or smaller,
   wherein said hydrophilic clay comprises laponite clay,
   wherein said quaternary ammonium compound comprises benzethonium chloride or derivatives thereof.

2. The composition of claim 1, wherein said suspension comprises 0.05% of said dispersion in a 1% glycerin in water solution.

3. The composition according to claim 1 or 2, wherein said dispersion further comprises an active agent, preferably wherein said active agent comprises one or more of: an antimicrobial, an analgesic, an anti-fungal, and an anti-inflammatory, and/or wherein said dispersion comprises one or more of a chelating agent, emulsifier, humectants, moisturizer, detackifier, emollient, and thickener.

4. The composition of any one of claims 1 to 3, wherein said suspension comprises particles of less than 0.45 micron in diameter.

5. A method of making a suspension of any one of claims 1 to 4, comprising the steps of:

   a) mixing a dispersion made by the method of i) dry mixing said hydrophilic clay and said quaternary ammonium compound to produce a mixture; ii) adding water to said mixture; and iii) incubating said mixture to form said dispersion; b) mixing said dispersion with glycerin; c) adding water to make a suspension; and d) filtering said suspension, preferably . wherein said suspension comprises 0.01% to 0.1% of said dispersion in a 0.5% to 5% glycerin in water solution, more preferably wherein said suspension comprises 0.05% of said dispersion in 1% glycerin.

6. A pharmaceutical composition comprising:

   a) the composition of any one of claims 1 to 4; and
   b) a pharmaceutically acceptable carrier.

7. A kit, comprising:

a) the composition of any one of claims 1 to 4 or 6; and
b) an administration device, preferably wherein said composition is provided in dosage form.

**8.** The composition of any one of claims 1 to 6, wherein said composition is formulated as an oral spray, an oral rinse, an ophthalmic drop, or a disinfectant.

**9.** A bandage or dressing comprising composition of any one of claims 1 to 4 or 6.

**10.** The composition according to any one of claims 1 to 4 or 6 for use as a medicament, the use comprising: administering the composition to a subject in need thereof, preferably wherein said administering is selected from the group consisting of oral administration, intravenous administration, intramuscular administration, subcutaneous administration, and topical administration.

**11.** The composition for use according to claim 10, wherein said subject is selected from the group consisting of a human, a companion animal, and a livestock animal.

**12.** The composition for use according to claim 10 or 11, wherein said composition treats an infection or condition in said subject, preferably wherein said condition is sepsis or wherein said composition adsorbs endotoxins.

## Patentansprüche

**1.** Zusammensetzung, umfassend

eine Suspension einer Dispersion in Glycerin, wobei die Dispersion eine dispergierte Phase innerhalb einer wässrigen Phase umfasst, und wobei die Dispersion Folgendes umfasst:

a) einen hydrophilen Ton in der dispergierten Phase; und
b) eine quaternäre Ammoniumverbindung in der dispergierten Phase,

wobei die Suspension 0,01 % bis 0,1 % der Dispersion in einer 0,5%igen bis 5%igen Glycerin in Wasser-Lösung umfasst,
wobei die Suspension filtriert ist, wobei der Filter 0,45 Mikrometer oder kleiner ist,
wobei der hydrophile Ton Laponitton umfasst,
wobei die quaternäre Ammoniumverbindung Benzethoniumchlorid oder Derivative davon umfasst.

**2.** Zusammensetzung nach Anspruch 1, wobei die Suspension 0,05 % der Dispersion in einer 1%igen Glycerin in Wasser-Lösung umfasst.

**3.** Zusammensetzung nach Anspruch 1 oder 2, wobei die Dispersion ferner einen Wirkstoff umfasst, wobei der Wirkstoff vorzugsweise eines oder mehreres von Folgendem umfasst: ein antimikrobielles Mittel, ein Analgetikum, ein antimykotisches Mittel und ein entzündungshemmendes Mittel, und/oder wobei die Dispersion eines oder mehrere aus einem Chelatbildner, Emulgator, Feuchthaltemittel, Befeuchtungsmittel, Antiklebemittel, Emolliens und Verdickungsmittel umfasst.

**4.** Zusammensetzung nach einem der Ansprüche 1 bis 3, wobei die Suspension Teilchen mit einem Durchmesser von weniger als 0,45 Mikrometer umfasst.

**5.** Verfahren zur Herstellung einer Suspension nach einem der Ansprüche 1 bis 4, umfassend die folgenden Schritte:

a) Mischen einer Dispersion, hergestellt nach Verfahren i) Trockenmischen des hydrophilen Tons und der quaternären Ammoniumverbindung, um ein Gemisch zu produzieren; ii) Hinzufügen von Wasser zu dem Gemisch; und iii) Inkubieren des Gemischs, um die Dispersion zu bilden; b) Mischen der Dispersion mit Glycerin; c) Hinzufügen von Wasser, um eine Suspension herzustellen; und d) Filtern der Suspension, wobei die Suspension vorzugsweise 0,01 % bis 0,1% der Dispersion in einer 0,5%igen bis 5%igen Glycerin in Wasser-Lösung umfasst, wobei die Suspension besonders bevorzugt 0,05 % % der Dispersion in 1 % Glycerin umfasst.

**6.** Pharmazeutische Zusammensetzung, umfassend:

a) die Zusammensetzung nach einem der Ansprüche 1 bis 4; und
b) einen pharmazeutisch unbedenklichen Träger.

**7.** Kit, umfassend:

a) die Zusammensetzung nach einem der Ansprüche 1 bis 4 oder 6; und
b) eine Verabreichungsvorrichtung, wobei die Zusammensetzung vorzugsweise in Dosierungsform vorliegt.

**8.** Zusammensetzung nach einem der Ansprüche 1 bis 6, wobei die Zusammensetzung als ein Mundspray, eine Mundspülung, Augentropfen oder ein Desinfektionsmittel formuliert wird.

**9.** Bandage oder Verband, umfassend Zusammensetzung nach einem der Ansprüche 1 bis 4 oder 6.

**10.** Zusammensetzung nach einem der Ansprüche 1 bis 4 oder 6 zur Verwendung als ein Arzneimittel, wobei die Verwendung Folgendes umfasst:
Verabreichung der Zusammensetzung an ein Individuum, das diese benötigt, wobei die Verabreichung vorzugsweise aus der Gruppe bestehend aus oraler Verabreichung, intravenöser Verabreichung, intramuskulärer Verabreichung, subkutaner Verabreichung und topischer Verabreichung ausgewählt wird.

**11.** Zusammensetzung zur Verwendung nach Anspruch 10, wobei das Individuum aus der Gruppe bestehend aus einem Menschen, einem Haustier und einem Nutztier ausgewählt wird.

**12.** Zusammensetzung zur Verwendung nach Anspruch 10 oder 11, wobei die Zusammensetzung eine Infektion oder einen Zustand des Individuums behandelt, wobei der Zustand vorzugsweise Sepsis ist oder wobei die Zusammensetzung Endotoxine adsorbiert.

## Revendications

**1.** Composition, comprenant :

une suspension d'une dispersion dans la glycérine, ladite dispersion comprenant une phase dispersée dans une phase aqueuse, et où la dispersion comprend :

a) une argile hydrophile dans ladite phase dispersée ; et
b) un composé d'ammonium quaternaire dans ladite phase dispersée,

dans laquelle ladite suspension comprend 0,01% à 0,1% de ladite dispersion dans une solution de 0,5% à 5% de glycérine dans l'eau,
dans laquelle ladite suspension est filtrée, où ledit filtre est de 0,45 micron ou moins,
dans laquelle ladite argile hydrophile comprend l'argile laponite,
dans laquelle ledit composé d'ammonium quaternaire comprend le chlorure de benzéthonium ou ses dérivés.

**2.** Composition de la revendication 1, dans laquelle ladite suspension comprend 0,05% de ladite dispersion dans une solution de 1% de glycérine dans l'eau.

**3.** Composition selon la revendication 1 ou 2, dans laquelle ladite dispersion comprend en outre un agent actif, de préférence dans laquelle ledit agent actif comprend un ou plusieurs parmi : un antimicrobien, un analgésique, un antifongique et un anti-inflammatoire, et/ou dans laquelle ladite dispersion comprend un ou plusieurs parmi un agent chélateur, un émulsifiant, des humectants, un hydratant, un antiadhésif, un émollient et un épaississant.

**4.** Composition de l'une quelconque des revendications 1 à 3, dans laquelle ladite suspension comprend des particules d'un diamètre inférieur à 0,45 micron.

**5.** Procédé de réalisation d'une suspension de l'une quelconque des revendications 1 à 4, comprenant les étapes :

a) de mélange d'une dispersion réalisée par le procédé i) de mélange à sec de ladite argile hydrophile et dudit composé d'ammonium quaternaire pour produire un mélange ; ii) d'ajout de l'eau audit mélange ; et iii)

d'incubation dudit mélange pour former ladite dispersion ; b) de mélange de ladite dispersion avec de la glycérine ; c) d'ajout de l'eau pour réaliser une suspension ; et d) de filtration de ladite suspension, de préférence où ladite suspension comprend 0,01% à 0,1% de ladite dispersion dans une solution de 0,5% à 5% de glycérine dans l'eau, plus préférablement où ladite suspension comprend 0,05% de ladite dispersion dans 1% de glycérine.

**6.** Composition pharmaceutique comprenant :

a) la composition de l'une quelconque des revendications 1 à 4 ; et
b) un support pharmaceutiquement acceptable.

**7.** Kit, comprenant :

a) la composition de l'une quelconque des revendications 1 à 4 ou 6 ; et
b) un dispositif d'administration, de préférence où ladite composition est fournie sous forme galénique.

**8.** Composition de l'une quelconque des revendications 1 à 6, dans laquelle ladite composition est formulée comme une pulvérisation orale, un rinçage oral, une goutte ophtalmique ou un désinfectant.

**9.** Bandage ou pansement comprenant la composition de l'une quelconque des revendications 1 à 4 ou 6.

**10.** Composition selon l'une quelconque des revendications 1 à 4 ou 6 pour une utilisation en tant que médicament, l'utilisation comprenant :
l'administration de la composition à un sujet en ayant besoin, de préférence où ladite administration est choisie dans le groupe constitué par une administration orale, une administration intraveineuse, une administration intramusculaire, une administration sous-cutanée et une administration topique.

**11.** Composition pour une utilisation selon la revendication 10, dans laquelle le sujet est choisi dans le groupe constitué par un humain, un animal de compagnie et un animal d'élevage.

**12.** Composition pour une utilisation selon la revendication 10 ou 11, dans laquelle ladite composition traite une infection ou une affection chez ledit sujet, de préférence dans laquelle ladite affection est une sepsie ou dans laquelle ladite composition adsorbe les endotoxines.

FIG. 1

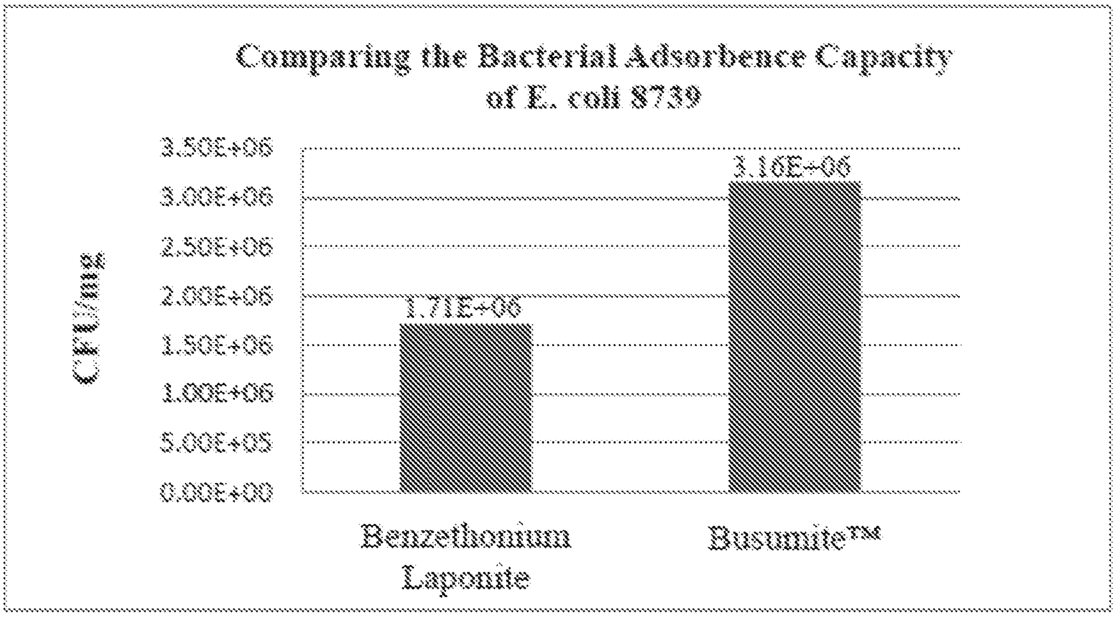
Comparing the Bacterial Adsorbence Capacity of E. coli 8739
CFU/mg
3.50E+06
3.00E+06
2.50E+06
2.00E+06
1.50E+06
1.00E+06
5.00E+05
0.00E+00
1.71E+06
3.16E+06
Benzethonium Laponite
Busumite™

FIG. 2

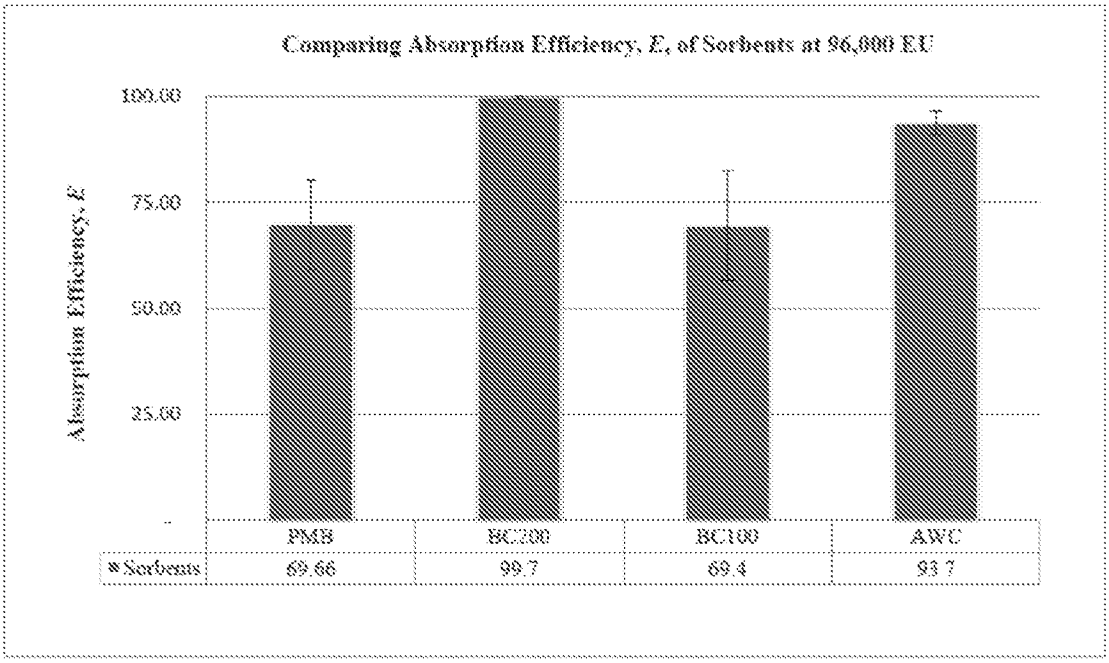
Comparing Absorption Efficiency, E, of Sorbents at 96,000 EU
Absorption Efficiency, E
100.00
75.00
50.00
25.00
-
PMB
BC200
BC100
AWC
Sorbents
69.66
99.7
69.4
93.7

FIG. 3

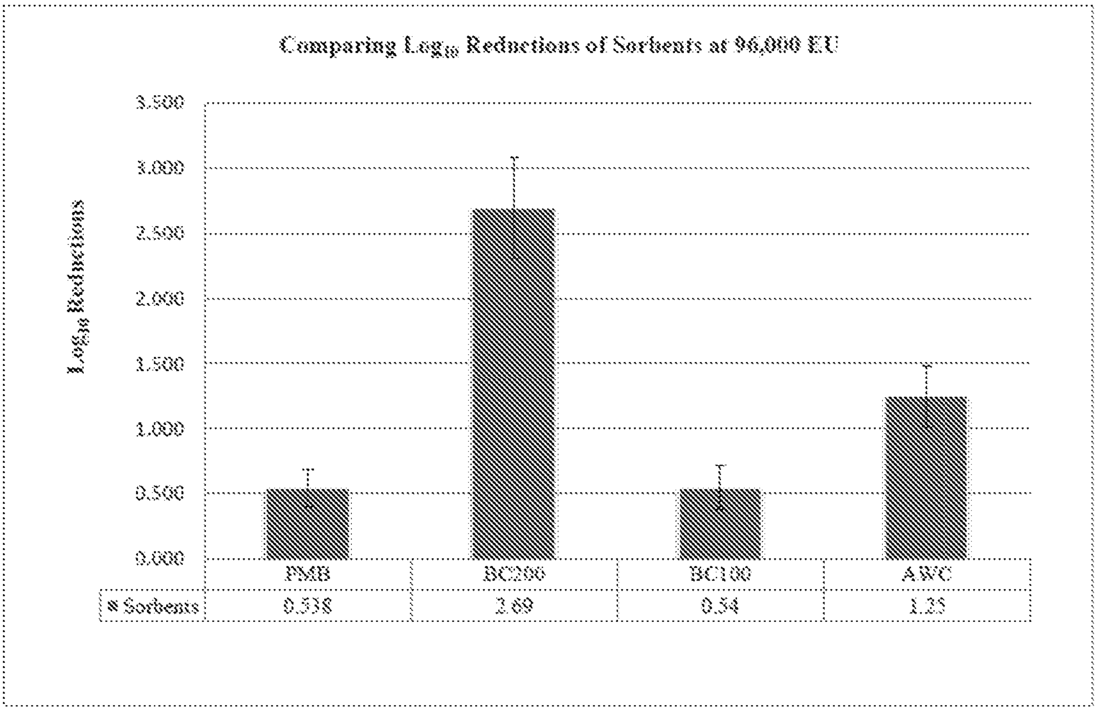
Comparing $Log_{10}$ Reductions of Sorbents at 96,000 EU
$Log_{10}$ Reductions
3.500
3.000
2.500
2.000
1.500
1.000
0.500
0.000
PMB
BC200
BC100
AWC
Sorbents
0.538
2.69
0.54
1.25

FIG. 4

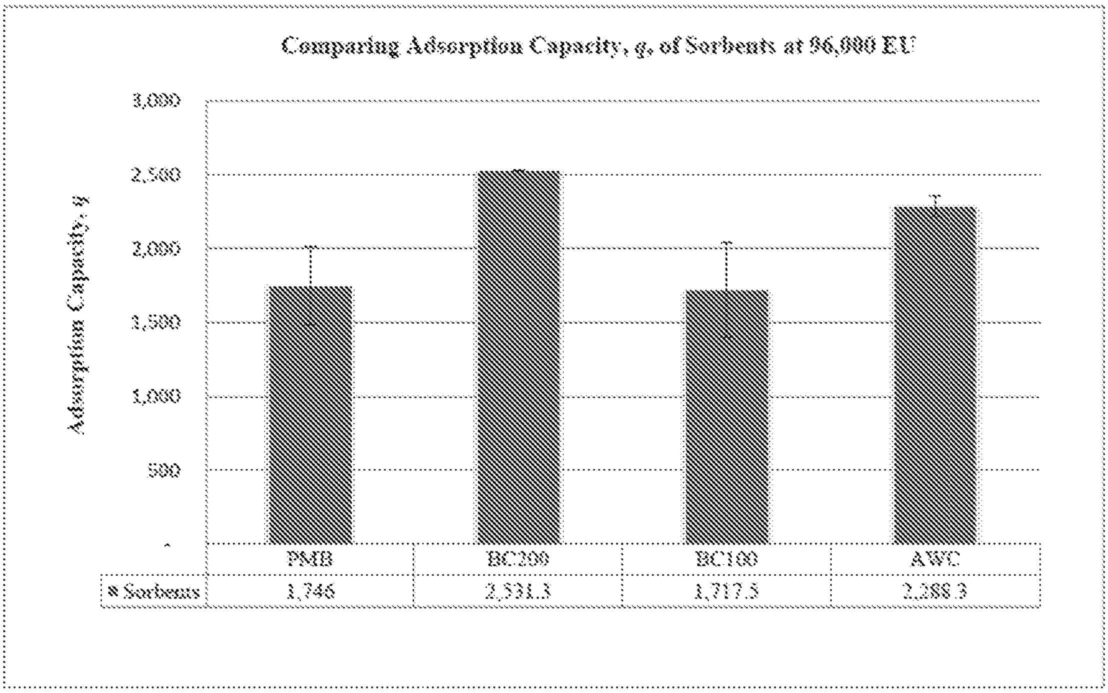
Comparing Adsorption Capacity, q, of Sorbents at 96,000 EU
Adsorption Capacity, q
3,000
2,500
2,000
1,500
1,000
500
-
PMB
BC200
BC100
AWC
Sorbents
1,746
2,531.3
1,717.5
2,288.3

FIG. 5

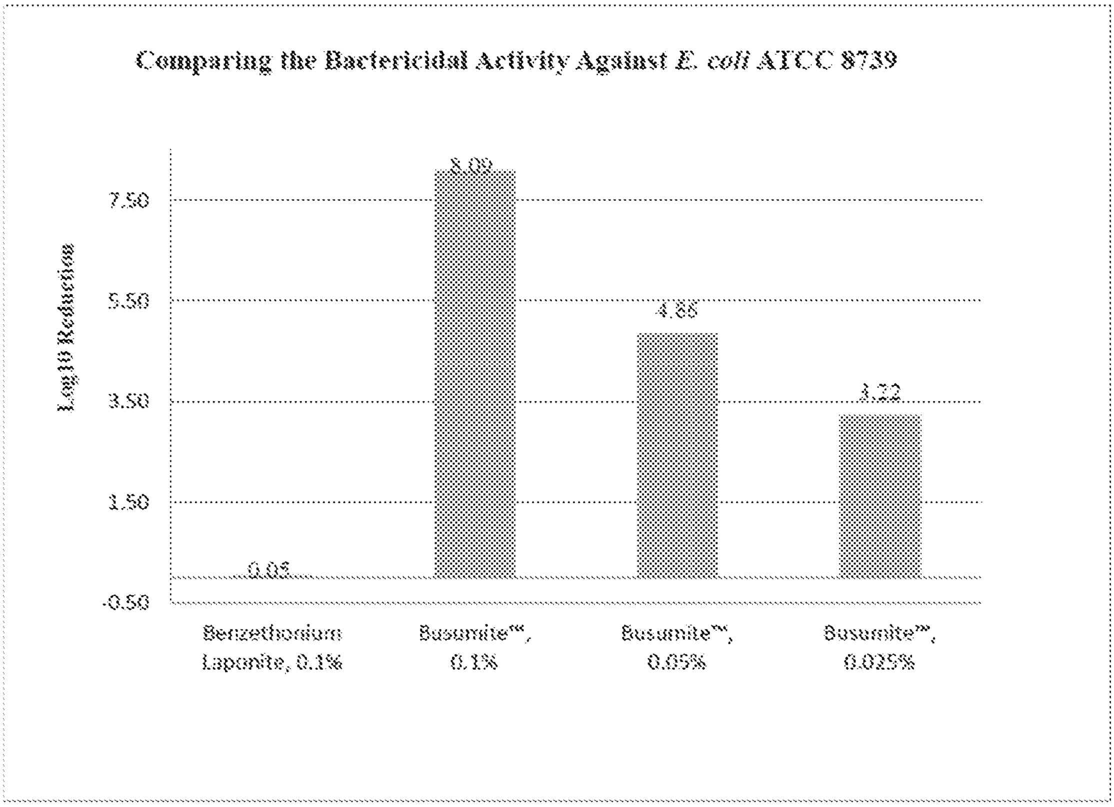
Comparing the Bactericidal Activity Against E. coli ATCC 8739
Log10 Reduction
7.50
5.50
3.50
1.50
-0.50
0.05
8.09
4.86
3.22
Benzethonium Laponite, 0.1%
Busumite™, 0.1%
Busumite™, 0.05%
Busumite™, 0.025%

FIG. 6

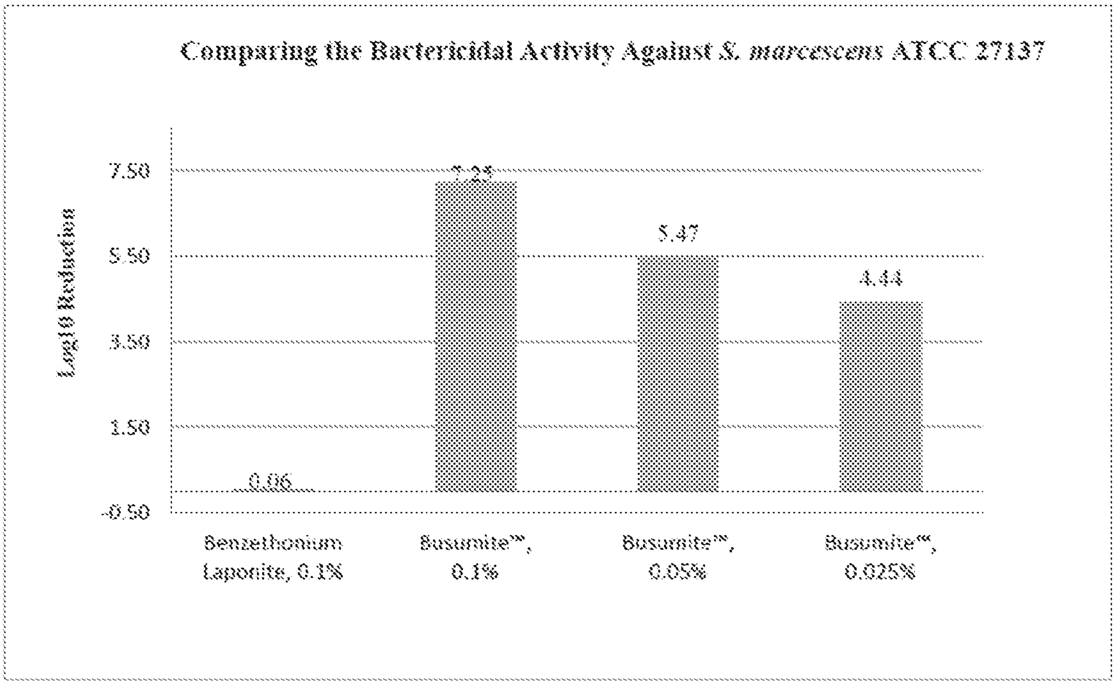
Comparing the Bactericidal Activity Against S. marcescens ATCC 27137
Log10 Reduction
7.50
5.50
3.50
1.50
-0.50
0.06
7.25
5.47
4.44
Benzethonium Laponite, 0.1%
Busumite™, 0.1%
Busumite™, 0.05%
Busumite™, 0.025%

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62746608
- US 2016000920 A1
- US 2016000920 A
- US 8858970 B
- US 6288076 B